# EUROPÄISCHE PATENTSCHRIFT

(11) **EP 0 700 886 B1**
(45) Veröffentlichungstag und Bekanntmachung des Hinweises auf die Patenterteilung: **26.04.2000**
(21) Anmeldenummer: 95113905.4
(22) Anmeldetag: 05.09.1995
(51) Int. Cl.: C07C 29/132, C07C 31/20

(54) **Verfahren zur Herstellung von 1,4-Butandiol**
Method for the preparation of 1,4-butanediol
Procédé pour la préparation de butane-1,4-diol

(30) Priorität: 06.09.1994 DE 4431743
(43) Veröffentlichungstag der Anmeldung: 13.03.1996
(73) Patentinhaber: BASF AKTIENGESELLSCHAFT, 67056 Ludwigshafen (DE)
(72) Erfinder: Fischer, Rolf, Dr., D-69121 Heidelberg (DE); Sigwart, Christoph, Dr., D-69198 Schriesheim (DE)
(74) Vertreter: Isenbruck, Günter, Dr.

(56) Entgegenhaltungen:
- EP-A- 0 610 600
- CHEMICAL ABSTRACTS, vol. 89, no. 24, 11.Dezember 1978 Columbus, Ohio, US; abstract no. 198249s, S. MABUCHI ET AL. 'Oxidation of 1,3-butadiene.' Seite 20; & JP-A-53 091 999 (TOYO SODA MFG. CO., LTD.)
- Houben-Weyl, Methoden der organischen Chemie, Band VI/3, 1965, Seiten 434-441
- K. Weissermel, H.-J. Arpe, "Industrielle Organische Chemie", 4.Aufl. VCH-Verlag, Seite 112
- J.Org.Chem. 1984, 49, 3707-3711
- Ullmann's Encyclopedia of Industrial Chemistry, 5.Aufl., Vol. A26, Seite 222
- Houben-Weyl, Methoden der organischen Chemie, Band VI/1a, 1979, Seiten 338 und 340

## Beschreibung

Die vorliegende Erfindung betrifft ein Verfahren zur Herstellung von 1,4-Butandiol durch Hydrierung von 1,3-Butadiendiepoxid.

1,4-Butandiol ist ein wichtiger, weltweit hergestellter chemischer Synthesebaustein; es dient beispielsweise als Ausgangsmaterial für die Synthese von Epoxidharzen, Polyestern, Polyamiden und Polyurethanen. Klassisch wird es durch Hydrierung von 2-Butin-1,4-diol hergestellt, welches wiederum das Produkt einer Reppe-Synthese aus Acetylen und Formaldehyd ist..

Die katalytische Hydrogenolyse von Oxiranen ist vielfach beschrieben worden. Eine Übersicht über die hydrogenolytische Spaltung von Oxiranen zu Alkoholen an verschiedenen Hydrierkatalysatoren ist beispielsweise in Houben-Weyl, Methoden der Organischen Chemie, Band VI, 1a, Teil 1, 1979, S. 338 bis 342, aufgeführt. Als gängige Katalysatoren für die hydrierende Oxiranspaltung werden Palladium-, Platin- und Nickel-Katalysatoren genannt.

In Houben-Weyl, Methoden der Organischen Chemie, Band VI, 3, 1965, S. 442 bis 446, wird die katalytische Hydrierung von unsymmetrischen 1,2-Epoxiden beschrieben. Als Regel für die Regioselektivität der Dreiringöffnung wird dabei angegeben, daß im allgemeinen zwischen dem Sauerstoffatom und demjenigen Kohlenstoffatom eine Spaltung erfolgt, das die geringste Zahl von Wasserstoffatomen trägt.

Die katalytische Hydrierung von Oxiranen unter Spaltung einer C-O-Bindung wird außerdem in Houben-Weyl, Methoden der Organischen Chemie, Band IV, 1c, 1980, S. 374 bis 377, beschrieben. Hier wird festgestellt, daß die Angaben über die Richtung der Ringöffnung bei unsymmetrisch substituierten Oxiranen in der Literatur zum Teil widersprüchlich sind. Trotzdem wird im Widerspruch zur im voranstehenden Absatz angegebenen Literaturstelle als Regel formuliert, daß bei fehlender Aktivierung durch Aryl- oder Allyl-Gruppen bei unsymmetrisch substituierten Oxiranen diejenige C-O-Bindung gespalten wird, die sterisch am wenigsten abgeschirmt ist, d. h. deren Kohlenstoffatom die höchste Zahl von Wasserstoffatomen trägt.

Die katalytische Hydrierung von 1,3-Butadiendiepoxid ist neu. Die widersprüchlichen Aussagen bezüglich der Selektivität bei der Ringspaltung in unsymmetrisch substituierten Oxiranen im Stand der Technik lassen dabei keine Vorhersage über das zu erwartende Produkt, also 1,4-, 2,3- oder 1,3-Butandiol, zu.

Aufgabe der Erfindung war es, einen Zugang zu 1,4-Butandiol zu schaffen, der die Reppe-Synthese mit ihren nicht unbedenklichen Katalysatoren vermeidet und dennoch wirtschaftlich ist.

Erfindungsgemäß wird diese Aufgabe durch ein Verfahren zur selektiven katalytischen Hydrierung von 1,3-Butadiendiepoxid zu 1,4-Butandiol gelöst, das dadurch gekennzeichnet ist, daß man 1,3-Butadiendiepoxid in Gegenwart von Wasserstoff an einem Hydrierkatalysator umsetzt, dessen aktiver Bestandteil eines oder mehrere der Elemente Kobalt, Nickel oder Rhenium ist und daß das Verfahren bei einer Temperatur zwischen 100 und 300°C, bevorzugt zwischen 100 und 250°C, besonders bevorzugt zwischen 100 und 220°C, durchgeführt wird.

In dem erfindungsgemäßen Verfahren erfolgt die überraschenderweise selektive Hydrogenolyse der Epoxidringe von 1,3-Butadiendiepoxid unter Bildung von 1,4-Butandiol gemäß der folgenden Reaktionsgleichung:

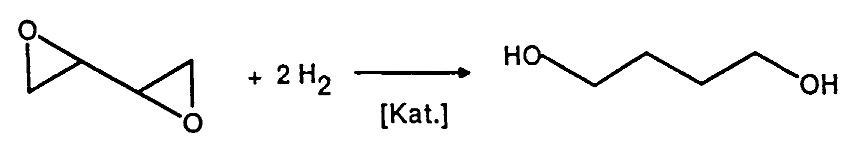

Bei der Durchführung des erfindungsgemäßen Verfahrens wird 1,3-Butadiendiepoxid in Gegenwart von Wasserstoff und eines wie oben definierten Hydrierkatalysators gewöhnlich bei einem Druck von 1 bis 300 bar, vorzugsweise 10 bis 250 bar und insbesondere 20 bis 200 bar und bei einer Temperatur von 100 bis 300°C, vorzugsweise 100 bis 250°C und besonders bevorzugt 100 bis 220°C zu 1,4-Butandiol umgesetzt.

Das erfindungsgemäße Verfahren kann ohne Lösungsmittel oder vorteilhaft in Anwesenheit eines unter den Reaktionsbedingungen inerten Lösungsmittels durchgeführt werden. Bei derartigen Lösungsmitteln kann es sich z.B. um Ether, wie beispielsweise Tetrahydrofuran, Methyl-tert.-butylether oder Di-n-butylether, Alkohole, wie beispielsweise Methanol, Ethanol, Propanol, i-Propanol, Butanol, i-Butanol oder tert.-Butanol, Kohlenwasserstoffe, wie beispielsweise Petrolether, und N-Alkyl-Lactame, wie beispielsweise N-Methylpyrrolidon oder N-Octylpyrrolidon, handeln.

Die Hydrierkatalysatoren können im erfindungsgemäßen Verfahren im allgemeinen in jeder zur Hydrogenolyse von Oxiranen geeigneten Form vorliegen. So kann es sich um homogen im Reaktionsmedium lösliche Hydrierkatalysatoren, wie sie beispielsweise in Houben-Weyl, Methoden der Organischen Chemie, Band IV/lc, Seiten 45 bis 67, beschrieben werden, handeln.

Vorzugsweise werden im erfindungsgemäßen Verfahren jedoch heterogene Hydrierkatalysatoren verwendet, also solche Hydrierkatalysatoren, die im Reaktionsmedium im wesentlichen unlöslich sind. Von diesen Hydrierkatalysatoren sind diejenigen geeignet, die ein oder mehrere der Elemente Rhenium, Kobalt und/oder Nickel, enthalten.

Im erfindungsgemäßen Verfahren können als heterogene Hydrierkatalysatoren solche verwendet werden, die aus Metallen in aktiver, feinverteilter Form mit großer Oberfläche bestehen, beispielsweise Raney-Nickel, Raney-Kobalt oder Rhenium-Schwamm.

Weiter können im erfindungsgemäßen Verfahren z. B. sogenannte Fällungskatalysatoren verwendet werden. Solche Katalysatoren können hergestellt werden, indem man ihre katalytisch aktiven Komponenten aus deren Salzlösungen, insbesondere aus den Lösungen von deren Nitraten und/oder Acetaten, beispielsweise durch Zugabe von Alkalimetall- und/oder Erdalkalimetallhydroxid- und/oder -carbonat-Lösungen, als beispielsweise schwerlösliche Hydroxide, Oxidhydrate, basische Salze oder Carbonate ausfällt, die erhaltenen Niederschläge anschließend trocknet und dann durch Calcinierung bei im allgemeinen 300 bis 700°C, insbesondere 400 bis 600°C, in die betreffenden Oxide, Mischoxide und/oder gemischt-wertigen Oxide umwandelt, welche durch eine Behandlung mit Wasserstoff oder mit wasserstoffhaltigen Gasen bei im allgemeinen 100 bis 700°C, insbesondere 150 bis 400°C, zu den betreffenden Metallen und/oder oxidischen Verbindungen niedriger Oxidationsstufe reduziert und somit in die eigentliche, katalytisch aktive Form überführt werden. Dabei wird in der Regel so lange reduziert, bis kein Wasser mehr gebildet wird.

Bei der Herstellung von Fällungskatalysatoren, die ein Trägermaterial enthalten, kann die Fällung der katalytisch aktiven Komponenten in Gegenwart des betreffenden Trägermaterials erfolgen. Die katalytisch aktiven Komponenten können vorteilhaft aber auch gleichzeitig mit dem Trägermaterial aus den betreffenden Salzlösungen gefällt werden.

Die Aktivierung sowohl der Fällungskatalysatoren als auch der Trägerkatalysatoren kann auch in situ in der Reaktionsmischung durch den dort anwesenden Wasserstoff erfolgen, bevorzugt erfolgt die Aktivierung jedoch gesondert vor der Verwendung.

Als Trägermaterialien können im allgemeinen die Oxide des Aluminiums und Titans, Zirkoniumdioxid, Siliciumdioxid, Kieselgur, Kieselgel, Tonerden, z. B. Montmorillonite, Silicate, wie Magnesium- oder Aluminiumsilicate, Zeolithe, wie ZSM-5 oder ZBM-10-Zeolithe, oder Aktivkohle verwendet werden. Bevorzugte Trägermaterialien sind Aluminiumoxide, Titandioxide, Siliciumdioxide, Zirkoniumdioxide und Aktivkohle. Selbstverständlich können auch Mischungen verschiedener Trägermaterialien als Träger für die im erfindungsgemäßen Verfahren anwendbare Katalysatoren dienen.

Das erfindungsgemäße Verfahren kann sowohl kontinuierlich als auch diskontinuierlich durchgeführt werden. Bei der kontinuierlichen Betriebsweise können beispielsweise Rohrreaktionen eingesetzt werden, in denen der Katalysator vorteilhaft in Form eines Festbetts angeordnet ist, über das die Reaktionsmischung in Sumpf- oder Rieselfahrweise geleitet werden kann. Bei der diskontinuierlichen Betriebsweise können sowohl einfache Rührreaktoren als auch vorteilhaft Schlaufenreaktoren verwendet werden. Bei Verwendung von Schlaufenreaktoren wird der Katalysator zweckmäßigerweise in Form eines Festbetts angeordnet. Das erfindungsgemäße Verfahren kann sowohl in der Gas- als auch in der Flüssigphase durchgeführt werden.

Das als Ausgangsmaterial verwendete 1,3-Butadiendiepoxid kann beispielsweise durch basisch induzierte Dehydrochlorierung von Dichlorbutandiolen nach Przem. Chem. 56 (1977) 5, 246-50 bzw. Dehydrobromierung von 1,4-Dibrombutandiol nach SU 1057-502-A hergestellt werden. Halogenfrei kann Butadiendiepoxid beispielsweise durch Epoxidierung von Butadien mit Wasserstoffperoxid an Titan-Silicaliten als Katalysator nach Chim. Ind. (Milan), 72 (7), 610-16 bzw. nach EP 100 119 A hergestellt werden. Weiter kann Butadiendiepoxid durch Epoxidierung von Butadien mit organischen Persäuren nach JP 61072774 A oder mit Cyclohexanonperoxid unter Verwendung von Titandioxid als Katalysator nach EP 129 814 hergestellt werden. Schließlich kann Butadien nach Zh. Obshch. Khim. 46 (6) 1976, 1420, mit Luft-Sauerstoff bei 250°C in einem Rohrreaktor zu Butadiendiepoxid oxidiert werden.

### Katalysatoren

Die in den Beispielen verwendeten Katalysatoren A bis D, sowie H und I weisen im nichtreduzierten Zustand die in Tabelle I angegebenen Zusammensetzungen auf (Angaben in Gew.-%):

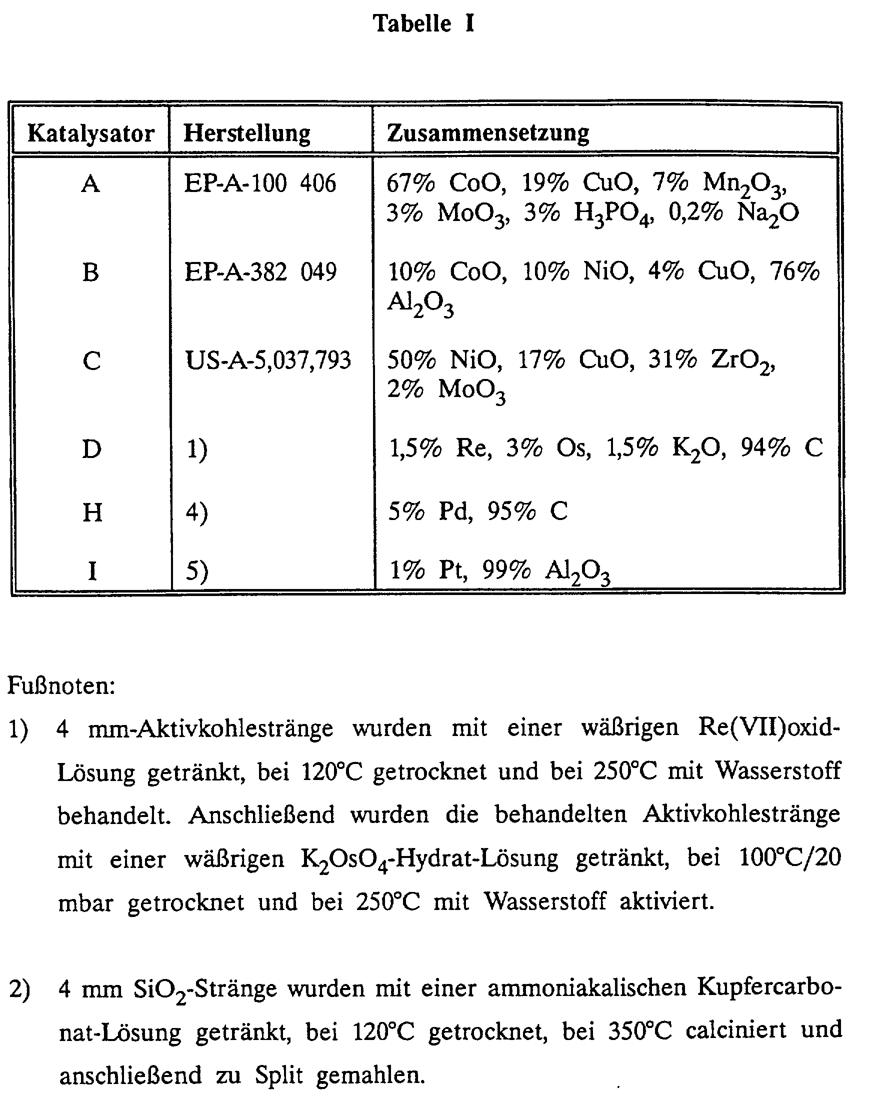

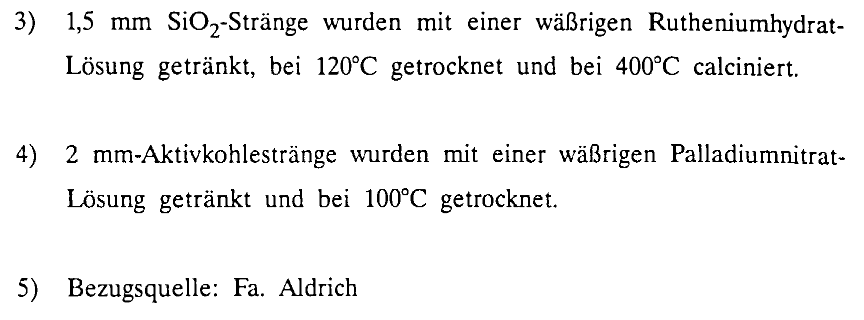

Die in den folgenden Beispielen verwendete Angabe Mol-% bezieht sich auf umgesetztes 1,3-Butadiendiepoxid.

### Beispiel 1

In einem 50 ml-Metallautoklaven wurde die zu hydrierende Lösung aus 2,5 g rac-1,3-Butadiendiepoxid und 22,5 g Tetrahydrofuran mit 0,5 g Raney-Kobalt versetzt und unter magnetischem Rühren 3 h bei 180°C und 40 bar mit Wasserstoff hydriert. Bei einem Umsatz von 98% wurden nach gaschromatographischer Analyse 80 Mol-% 1,4-Butandiol und 19 Mol-% n-Butanol erhalten.

### Beispiel 2

Die Hydrierung von 2,5 g 1,3-Butadiendiepoxid und 22,5 g Tetrahydrofuran wurde wie in Beispiel 1, jedoch an Katalysator A anstelle von Raney-Kobalt, durchgeführt. Katalysator A wurde dabei zunächst im Wasserstoffstrom während 2 h bei 250°C aktiviert und anschließend in Form von 4-mm-Strängen eingesetzt. Bei quantitativem Umsatz wurden 81 Mol-% 1,4-Butandiol, 16 Mol-% n-Butanol und 3 Mol-% 2,3-Butandiol gefunden.

### Beispiel 3

Die Hydrierung von 2,5 g 1,3-Butadiendiepoxid und 22,5 g Tetrahydrofuran mit 0,5 g Katalysator B erfolgte wie in Beispiel 1 beschrieben. Katalysator B wurde zuvor 1 h bei 250°C im Wasserstoffstrom aktiviert und in Form von 4-mm-Strängen eingesetzt. Bei einem Umsatz von 100% wurden 77 Mol-% 1,4-Butandiol und 8 Mol-% n-Butanol erhalten.

### Beispiel 4

Die Hydrierung von 2,5 g 1,3-Butadiendiepoxid und 22,5 g Tetrahydrofuran mit 0,5 g Raney-Nickel wurde bei 140°C und 40 bar in einer wie in Beispiel 1 beschriebenen Apparatur durchgeführt. Der Reaktionsaustrag enthielt nach einer Reaktionszeit von 4 h bei quantitativem Umsatz 74 Mol-% 1,4-Butandiol und 23 Mol-% 2,3-Butandiol.

### Beispiele 5 und 6

Die Reaktionsbedingungen und die Zusammensetzung der Reaktionsausträge, die bei der Hydrierung von 2,5 g 1,3-Butadiendiepoxid und 22,5 g Tetrahydrofuran an jeweils 0,5 g der Katalysatoren C und D bei 40 bar Gesamtdruck erzielt wurden, sind in der folgenden Tabelle II aufgeführt.

Katalysator C wurde vorher im Wasserstoffstrom während 2 h bei 200°C aktiviert und anschließend in Form von 3-mm-Tabletten eingesetzt.

Katalysator D war vorher durch Aktivierung im Wasserstoffstrom während 1 h bei 150°C hergestellt worden und wurde in Form von 4-mm-Strängen eingesetzt.

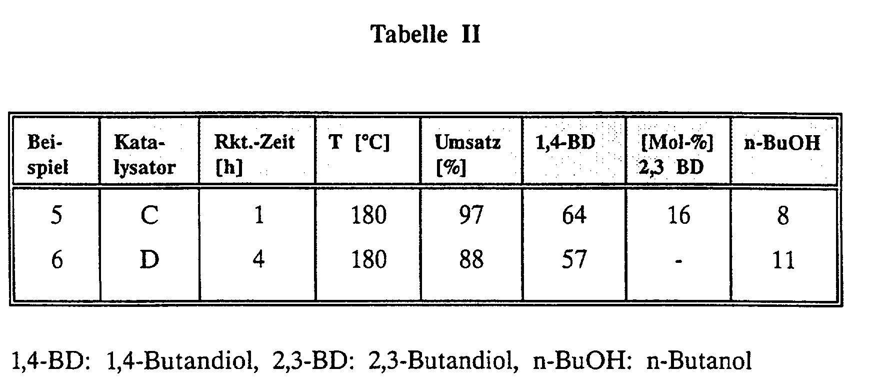

### Vergleichsbeispiel 1

Die Hydrierung von 2,5 g 1,3-Butadiendiepoxid und 22,5 g Tetrahydrofuran wurde mit 0,5 g Katalysator H bei 100°C und 40 bar in einer wie in Beispiel 1 beschriebenen Apparatur durchgeführt. Der Katalysator wurde zuvor 1 h bei 120°C im Wasserstoffstrom aktiviert und in Form von 2-mm-Strängen eingesetzt. Der Reaktionsaustrag enthielt nach einer Reaktionszeit von 4 h bei quantitativem Umsatz 7 Mol-% 1,4-Butandiol und 82 Mol-% 2,3-Butandiol.

### Vergleichsbeispiel 2

Die Hydrierung von 2,5 g 1,3-Butadiendiepoxid und 22,5 g Tetrahydrofuran wurde mit 0,5 g Katalysator I bei 180°C und 40 bar in einer wie in Beispiel 1 beschriebenen Apparatur durchgeführt. Der Katalysator wurde ohne Aktivierung in Pulverform eingesetzt. Der Reaktionsaustrag enthielt nach einer Reaktionszeit von 4 h bei einem Umsatz von 73% 1 Mol-% 1,4-Butandiol und 73 Mol-% 2,3-Butandiol.

## Patentansprüche

1. Verfahren zur Herstellung von 1,4-Butandiol, dadurch gekennzeichnet, daß man 1,3-Butadiendiepoxid in Gegenwart von Wasserstoff an einem Hydrierkatalysator umsetzt, dessen aktiver Bestandteil eines oder mehrere der Elemente Kobalt, Nickel oder Rhenium ist und daß das Verfahren bei einer Temperatur zwischen 100 und 300°C, bevorzugt zwischen 100 und 250°C, besonders bevorzugt zwischen 100 und 220°C, durchgeführt wird.

2. Verfahren nach Anspruch 1, dadurch gekennzeichnet, daß es sich bei dem Hydrierkatalysator um einen heterogenen Hydrierkatalysator handelt.

3. Verfahren nach Anspruch 1 oder 2, dadurch gekennzeichnet, daß der Hydrierkatalysator einen der folgenden Träger umfaßt: Oxide des Aluminiums und Titans, Zirkoniumdioxid, Siliciumdioxid, Kieselgur, Kieselgel, Tonerden, Silicate, Zeolithe, Aktivkohle.

4. Verfahren nach einem der Ansprüche 1 bis 3, dadurch gekennzeichnet, daß es in einem Lösungsmittel durchgeführt wird.

5. Verfahren nach einem der Ansprüche 1 bis 4, dadurch gekennzeichnet, daß man einen Hydrierkatalysator verwendet, der Kobalt enthält.

6. Verfahren nach einem der Ansprüche 1 bis 4, dadurch gekennzeichnet, daß man einen Hydrierkatalysator verwendet, der Nickel enthält.

7. Verfahren nach einem der Ansprüche 1 bis 4, dadurch gekennzeichnet, daß man einen Hydrierkatalysator verwendet, der Rhenium enthält.

## Claims

1. A process for the preparation of 1,4-butanediol, which comprises converting 1,3-butadiene diepoxide in the presence of hydrogen over a hydrogenation catalyst whose active component is one or more of the elements cobalt, nickel or rhenium and carrying out the process at from 100 to 300°C, preferably from 100 to 250°C, particularly preferably from 100 to 220°C.

2. A process as claimed in claim 1, wherein the hydrogenation catalyst is a heterogeneous hydrogenation catalyst.

3. A process as claimed in claim 1 or 2, wherein the hydrogenation catalyst comprises one of the following carriers: oxides of aluminum and of titanium, zirconium dioxide, silica, kieselguhr, silica gel, aluminas, silicates, zeolites and active carbon.

4. A process as claimed in any of claims 1 to 3, which is carried out in a solvent.

5. A process as claimed in any of claims 1 to 4, wherein a hydrogenation catalyst which contains cobalt is used.

6. A process as claimed in any of claims 1 to 4, wherein a hydrogenation catalyst which contains nickel is used.

7. A process as claimed in any of claims 1 to 4, wherein a hydrogenation catalyst which contains rhenium is used.

## Revendications

1. Procédé de préparation de 1,4-butanediol, caractérisé en ce qu'on fait réagir du diépoxy-1,3-butadiène en présence d'hydrogène sur un catalyseur d'hydrogénation dont le constituant actif est un ou plusieurs des éléments cobalt, nickel ou rhénium, et en ce que le procédé est mis en oeuvre à une température de 100 à 300°C, de préférence de 100 à 250°C et tout particulièrement de 100 à 220°C.

2. Procédé selon la revendication 1, caractérisé en ce que, pour ce qui concerne le catalyseur d'hydrogénation, il s'agit d'un catalyseur d'hydrogénation hétérogène.

3. Procédé selon la revendication 1 ou 2, caractérisé en ce que le catalyseur d'hydrogénation comporte l'un des supports suivants : oxydes de l'aluminium et du titane, dioxyde de zirconium, dioxyde de silicium, kieselguhr, gel de silice, alumines, silicates, zéolites, charbon actif.

4. Procédé selon l'une des revendications 1 à 3, caractérisé en ce qu'il est mis en oeuvre dans un solvant.

5. Procédé selon l'une des revendications 1 à 4, caractérisé en ce qu'on utilise un catalyseur d'hydrogénation contenant du cobalt.

6. Procédé selon l'une des revendications 1 à 4, caractérisé en ce qu'on utilise un catalyseur d'hydrogénation contenant du nickel.

7. Procédé selon l'une des revendications 1 à 4, caractérisé en ce qu'on utilise un catalyseur d'hydrogénation contenant du rhénium.
